# FASCICULE DE BREVET EUROPEEN

(11) **EP 0 685 227 B1**
(45) Date de publication et mention de la délivrance du brevet: **25.09.1996**
(21) Numéro de dépôt: 95401062.5
(22) Date de dépôt: 05.05.1995
(51) Int. Cl.: A61K 7/42, A61K 7/48, A61K 7/06

(54) **Compositions cosmétiques photoprotectrices contenant un système filtrant les rayons UV et des polymères particuliers et utilisations**
Kosmetisches Lichtschutzmittel enthaltend einen UV Filter und einen Polymer und seine Verwendung
Cosmetic photoprotection composition containing an ultraviolet filter and a polymer and uses thereof

(30) Priorité: 03.06.1994 FR 9406836
(43) Date de publication de la demande: 06.12.1995
(73) Titulaire: L'OREAL, F-75008 Paris (FR)
(72) Inventeur: Ascione, Jean-Marc, F-75018 Paris (FR); Allard, Delphine, F-92700 Colombes (FR); Hansenne, Isabelle, F-75017 Paris (FR)
(74) Mandataire: Andral, Christophe André Louis

(56) Documents cités:
- EP-A- 0 427 411
- FR-A- 2 398 496
- COSMETICS & TOILETRIES. EDIZIONE ITALIANA., vol.3, Juin 1994, MILANO (ITALY) pages 18 - 24 G. PROSERPIO 'Tecnica di Formulazione di Prodotti Cosmetici con l'impiego di: Acrysol 33.'

## Description

La présente invention concerne de nouvelles compositions cosmétiques améliorées à usage topique plus particulièrement destinées à la photoprotection de la peau et/ou des cheveux contre le rayonnement ultraviolet (compositions ci-après dénommées plus simplement compositions antisolaires), ainsi que leur utilisation dans l'application cosmétique susmentionnée. Plus précisément encore, elle concerne des compositions antisolaires à pouvoir photoprotecteur amélioré comprenant, dans un support cosmétiquement acceptable à phase aqueuse continue et en particulier de type émulsion huile-dans-eau, une association entre (i) un système photoprotecteur classique capable de filtrer les rayons UV (UV-A et/ou UV-B) et qui peut donc être constitué par un ou plusieurs filtres organiques solaires (absorbeurs d'UV) et/ou par un ou plusieurs (nano)pigments minéraux à base d'oxydes métalliques, et en particulier d'oxyde de titane, agissant par blocage physique du rayonnement (réflecteurs et/ou diffuseurs d'UV), et (ii) un ou plusieurs polymères particuliers, convenablement sélectionnés, et tels que définis ci-après.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber selectivement les rayonnements UV nocifs, ces filtres (et leurs quantités) étant sélectionnés en fonction de l'indice de protection recherché (l'indice de protection (IP) s'exprimant mathématiquement par le rapport du temps d'irradiation nécessaire pour atteindre le seuil érythématogène avec le filtre UV au temps nécessaire pour atteindre le seuil érythématogène sans filtre UV). Par ailleurs, l'utilisation de pigments ou de nanopigments minéraux (on entend par "nanopigments" des pigments dont la taille moyenne des particules primaires n'excède généralement pas 100 nm, cette taille étant de préférence comprise entre 5 nm et 100 nm, et plus préférentiellement encore comprise entre 10 et 50 nm) à base d'oxydes métalliques, et en particulier d'oxyde de titane, dans le domaine de la protection solaire, est également aujourd'hui courante, et l'on sait en particulier que ces substances, qu'elles soient ou non associées avec des filtres organiques usuels absorbeurs d'UV-A et/ou UV-B, sont capables d'apporter aux compositions antisolaires qui les contiennent un certain pouvoir photoprotecteur propre ou complémentaire, toutefois assez limité, et ceci en agissant par simple blocage physique des rayons UV (mécanismes de réflection et/ou diffusion du rayonnement).

Dans le but d'améliorer les propriétés essentiellement cosmétiques des compositions antisolaires, et plus particulièrement de celles décrites ci-dessus, il est par ailleurs maintenant usuel d'introduire dans ces dernières des polymères dits émulsionnants et parmi lesquels on peut tout particulièrement citer les copolymères réticulés de type Acide acrylique/Acrylates d'alkyles en C10-C30, tels que ceux connus sous les noms de marque "PEMULEN TR-1" et "CARBOPOL 1342" de chez Goodrich, dont l'emploi est en fait aujourd'hui des plus répandu (voir par exemple EP-A-0 427 411).

Toutefois, l'un des problèmes liés à la plupart des compositions cosmétiques antisolaires qui contiennent de tels polymères, qu'elles soient à base de filtres organiques ou de (nano)pigments ou bien encore d'un mélange entre ceux-ci, est que les indices de protection sur peau qui leur sont attachés peuvent, et en particulier pour les peaux dites très sensibles et/ou continuellement exposées au soleil, apparaitre comme encore insuffisants, et il serait donc utile, à cet égard, de pouvoir encore améliorer les propriétés photoprotectrices de ces compositions de l'art antérieur, tout en en préservant bien entendu les bonnes propriétés cosmétiques.

La présente invention vise à la satisfaction d'un tel besoin.

Or, à la suite d'importantes recherches menées dans le domaine de la photoprotection évoqué ci-dessus, la Demanderesse a maintenant découvert, de façon inattendue et surprenante, qu'il est possible d'obtenir des compositions antisolaires présentant à la fois de bonnes propriétés cosmétiques et des indices de protection supérieurs à ceux attachés aux compositions de l'art antérieur contenant des polymères émulsionnants (à concentration égale de filtre(s) et/ou de (nano)pigments et à nature identique de support) en associant au système filtrant qu'elles contiennent un ou plusieurs polymères particuliers, convenablement sélectionnés, et tels que définis ci-dessous.

Cette découverte, d'application très générale, est à la base de la présente invention.

Ainsi, conformément à l'un des objets de la présente invention, il est maintenant proposé de nouvelles compositions cosmétiques, en particulier antisolaires, du type comprenant, dans un support cosmétiquement acceptable dont la phase continue est aqueuse, un système photoprotecteur capable de filtrer les rayons UV (UV-A et/ou UV-B) et au moins un polymère, et qui sont essentiellement caractérisées par le fait que ledit polymère est choisi au sein des polymères constitués par la répétition (i) d'un motif de formule (I) et (ii) d'un motif de formule (II) et/ou de formule (III) ci-dessous : 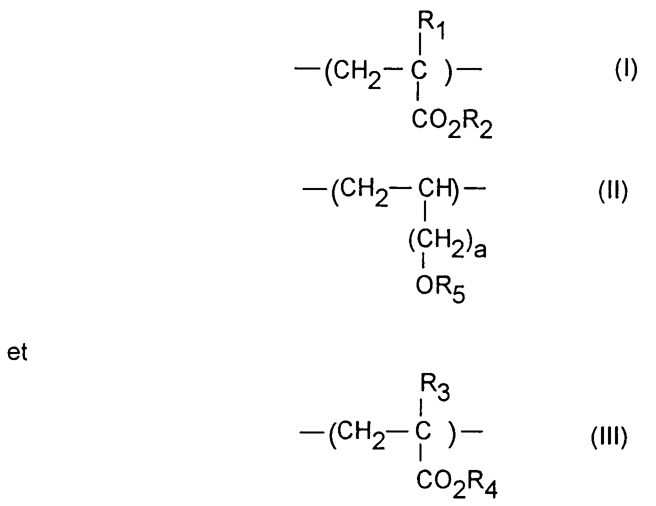
dans lesquelles a est un nombre entier égal à 0 ou 1, R₁, R₂, R₃ et R₄, qui peuvent être identiques ou différents, désignent l'hydrogène ou un radical alkyle en C₁-C₄, et R₅ désigne un radical CH₃CO- ou un radical R₆-(OC₂H₅)_{b}- dans lequel R₆ est un radical alkyle en C₂-C₂₀ et b un nombre entier compris inclusivement entre 1 et 20, étant entendu que lorsque ledit polymère est exempt de motif de formule (II) alors les radicaux R₂ et R₄ ne peuvent représenter simultanément l'hydrogène.

Selon l'invention, on entend désigner de manière générale par système photoprotecteur capable de filtrer le rayonnement UV, tout composé ou toute association de composés qui, par des mécanismes connus en soi d'absorption et/ou de réflection et/ou diffusion du rayonnement UV-A et/ou UV-B, permet d'empécher, ou du moins limiter, la mise en contact dudit rayonnement avec une surface (peau, cheveux,) sur laquelle ce ou ces composés ont été appliqués. En d'autres termes, les composés visés par la présente invention sont à la fois les filtres organiques photoprotecteurs absorbeurs d'UV et les (nano)pigments minéraux diffuseurs et/ou réflecteurs d'UV, ainsi que leurs mélanges

La présente invention a également pour objet l'utilisation des compositions ci-dessus comme, ou pour la fabrication de, compositions cosmétiques destinées à la protection de la peau et/ou des cheveux contre le rayonnement ultraviolet, en particulier le rayonnement solaire.

Un autre objet encore de la présente invention réside dans un procédé de traitement cosmétique pour la protection de la peau et/ou des cheveux contre le rayonnement ultraviolet, en particulier le rayonnement solaire, et qui consiste essentiellement à appliquer sur ces derniers une quantité efficace d'une composition conforme à l'invention.

D'autres caractéristiques, aspects et avantages de la présente invention apparaitront à la lecture de la description détaillée qui va suivre.

Les polymères particuliers utilisés, seuls ou en mélanges, dans le cadre de la présente invention sont classiquement obtenus par polymérisation du ou des monomères correspondant au(x) motif(s) désiré(s) dans le polymère final. Ainsi, tous les monomères utilisés pour la synthèse des polymères conformes à l'invention sont choisis au sein des familles des acides (alkyl)acryliques, des acrylates d'alkyles, des alcanoates de vinyle et des éthers ou esters allylique ou vinylique. Comme cela ressort de la définition donnée précédemment, les polymères conformes à la présente invention peuvent correspondre soit (i) à des copolymères (polymérisation de deux monomères différents) possédant simultanément le motif de formule (I) et le motif de formule (II), ou encore à des copolymères possédant simultanément le motif de formule (I) et le motif de formule (III), soit (ii) à des terpolymères (polymérisation de trois monomères différents) possédant simultanément le motif de formule (I), le motif de formule (II) et le motif de formule (III). Lorsque le polymère comprend un ou des motifs à fonction carboxylique, ce ou ces derniers peuvent être ramenés, lorsque cela est nécessaire, sous une forme partiellement ou totalement neutralisée, en particulier par réaction avec des agents alcalins et/ou en jouant sur le pH des compositions.

Selon un mode préféré de réalisation de la présente invention, les polymères mis en oeuvre sont des polymères réticulés, partiellement ou totalement.

En outre, toujours selon un mode préféré de réalisation de la présente invention, on ne retient des polymères ci-dessus que ceux qui sont capables de conférer à un milieu aqueux dans lequel ils sont introduits une viscosité d'au moins 5 poises, et ceci lorsqu'ils sont utilisés à des concentrations comprises entre 0,2 et 2 % en poids.

Les polymères encore plus particulièrement préférés dans le cadre de la présente invention sont :
- les terpolymères réticulés : Acide méthacrylique / Acrylate d'éthyle / Steareth-10 allyl ether ; de tels produits sont notamment vendus sous la dénomination commerciale de "SALCARE SC 90" par la Société Allied Colloids, et sont présentés sous la forme d'une émulsion aqueuse à 30 % MA environ.
- les copolymères réticulés : Acide acrylique / Acétate de vinyle ; de tels produits sont notamment vendus sous la dénomination commerciale de "RHEOLATE 5000" par la Société Rhéox.
- les copolymères réticulés : Acide acrylique / Acrylate d'éthyle ; de tels produits sont notamment vendus sous la dénomination commerciale de "ACRYSOL 33" par la Société Rohm et Haas, et sont présentés sous la forme d'une dispersion aqueuse à 30 % MA environ.

Les polymères conformes à l'invention sont généralement présents dans les compositions antisolaires selon l'invention à une concentration (exprimée en matière active MA) comprise entre 0,05 et 15 % en poids, et de préférence entre 0,1 et 4 % en poids, par rapport au poids total de la composition.

Comme indiqué précédemment, les polymères selon l'invention peuvent être associés soit à un ou plusieurs filtres organiques absorbeurs d'UV, soit à un ou plusieurs (nano)pigments minéraux, soit encore à des mélanges entre ceux-ci (système filtrant).

Les oxydes métalliques constitutifs des pigments ou des nanopigments utilisables dans le cadre des compositions antisolaires conformes à la présente invention peuvent être tous ceux déja connus en soi pour leur activité photoprotectrice. Ainsi, ils peuvent être notamment choisis, seuls ou en mélanges, parmi les oxydes de titane, de zinc, de fer, de zirconium, de cérium ou leurs mélanges.

De préférence, on met en oeuvre des nanopigments d'oxydes métalliques.

De tels nanopigments d'oxydes métalliques, enrobés ou non enrobés, sont des produits connus de l'homme de l'art et sont en particulier décrits dans la demande de brevet EP-A- 0 518 773, dont l'enseignement est, à cet égard, inclus à titre de référence dans la présente description. A titre de nanopigments commerciaux complémentaires non cités dans la demande précitée mais également utilisables dans le cadre de la présente invention, on peut en outre mentionner les produits vendus sous les noms de marque UVT M 160, UVT M 212 et UVT M 262 par la Société KEMIRA, et MT 100 SA ou MT 100 SAS par la Société TAYCA.

Comme indiqué précédemment, la taille moyenne des particules primaires des nanopigments qui peuvent être présents dans les compositions selon l'invention est généralement comprise entre 5 nm et 100 nm, de préférence entre 10 et 50 nm.

Selon un mode préféré de réalisation des compositions antisolaires selon l'invention, on fait appel à des nanopigments minéraux à base d'oxyde de titane. Cet oxyde de titane peut se présenter sous une forme cristallisée de type rutile et/ou anatase, et/ou sous une forme amorphe ou substantiellemnt amorphe. Comme indiqué précédemment, ce pigment peut être alors enrobé ou non enrobé, mais de préférence on utilise des pigments enrobés, par exemple par de l'alumine et/ou du stéarate d'aluminium.

Les nanopigments sont généralement présents dans les compositions selon l'invention à une concentration comprise entre 0,1 et 30 % en poids, et de préférence entre 1 et 20 % en poids, par rapport au poids total de la composition.

De même, les compositions antisolaires conformes à l'invention peuvent par ailleurs contenir un ou plusieurs filtres solaires organiques classiques (absorbeurs), actifs dans l'UV-A et/ou l'UV-B, hydrophiles ou lipophiles. A titre d'exemples, ces filtres peuvent être choisis, seuls ou en mélanges, parmi l'acide 2-phényl benzimidazole 5-sulfonique et ses sels, les dérivés cinnamiques tels que par exemple le p-méthoxycinnamate de 2-éthylhexyle, les dérivés salicyliques comme par exemple le salicylate de 2-éthylhexyle et le salicylate d'homomenthyle, les dérivés du camphre comme par exemple le 3(4-méthylbenzylidène)camphre ou l'acide camphosulfonique (1,4 divinylbenzène), les dérivés de triazine tels que la 2,4,6-tris [p-(2'-éthylhexyl-1'-oxycarbonyl)anilino] 1,3,5-triazine, les dérivés de la benzophénone tels que la 2-hydroxy 4-méthoxybenzophénone, les dérivés du dibenzoylméthane tels que le 4-tert-butyl 4'-méthoxydibenzoylméthane, les dérivés de β,β-diphénylacrylate tels que le α-cyano-β,β-diphénylacrylate de 2-éthylhexyle, les dérivés de l'acide p-aminobenzoïque comme par exemple le paradiméthylaminobenzoate d'octyle, l'anthranilate de menthyle, les polymères filtres et silicones filtres décrits dans la demande WO-93-04665. D'autres exemples de filtres organiques sont donnés dans la demande de brevet EP-A 0 487 404.

Le ou les filtres organiques sont généralement présents dans les compositions selon l'invention à une concentration comprise entre 0,1 et 30 % en poids, et de préférence entre 0,5 et 25 % en poids, par rapport au poids total de la composition.

De préférence, la teneur global du système filtrant (filtre(s) organique(s) + (nano)pigment(s)) n'excède pas 40 % du poids total de la composition antisolaire finale

Selon un mode préféré de réalisation de la présente invention, le support cosmétiquement acceptable dans lequel se trouvent contenus le système filtrant et le ou les polymères est une émulsion de type huile-dans-eau. Toutefois, l'utilisation de tout autre support dans lequel la phase continue se trouverait être effectivement une phase aqueuse (cas d'un simple gel aqueux par exemple) n'est pas exclue.

Les compositions selon l'invention peuvent également contenir des agents de bronzage et/ou de brunissage artificiels de la peau (agents autobronzants), tels que par exemple de la dihydroxyacétone (DHA).

Les compositions de l'invention peuvent comprendre en outre des adjuvants cosmétiques classiques notamment choisis parmi les corps gras (en particulier pour solubiliser les éventuels filtres organiques lipophiles), les solvants organiques, les épaississants ioniques ou non ioniques, les adoucissants, les antioxydants et notamment les antioxydants ARL, les opacifiants, les stabilisants, les émollients, les silicones , les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions de type huile-dans-eau.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Les compositions de l'invention peuvent être préparées selon les techniques bien connues de l'homme de l'art, en particulier celles destinées à la préparation d'émulsions de type huile-dans-eau.

Ces compositions peuvent se présenter en particulier sous la forme d'une crème, d'un lait, d'un gel ou d'un gel crème, et éventuellement être conditionnées en aérosol et se présenter sous forme de mousse ou de spray.

Lorsqu'il s'agit d'une émulsion de type huile-dans-eau, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Les compositions cosmétiques de l'invention peuvent être utilisées comme composition protectrice de l'épiderme humain ou des cheveux contre les rayons ultraviolets, comme composition antisolaire ou encore comme produit de maquillage (peau, cils, sourcils, etc...).

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Des exemples concrets, mais nullement limitatifs, illustrant l'invention, vont maintenant être donnés.

### EXEMPLES

On a préparé différentes formulations antisolaires se présentant sous la forme d'une émulsion de type huile-dans-eau et contenant (les quantités sont exprimées en % poids par rapport au poids total de la composition) :

| | |
|---|---|
| - Nanopigments de TiO₂ ("MT 100 T" de chez TAYCA) (taille moyenne des particules primaires : 15 nm) | 0 ou 5 % |
| - p-méthoxycinnamate de 2-éthylhexyle ("PARSOL MCX" de chez Givaudan)(filtre UV organique) | 0 ou 5 % |
| - Polymère (conforme et non conforme à l'invention) | x % MA |
| - Mélange monostéarate de glycérol/stéarate de polyéthylèneglycol à 100 moles d'OE ("Arlacel 165" d'ICI) | 20 % |
| - Acide stéarique | 4 % |
| - Alcool stéarylique | 1 % |
| - Vaseline Codex | 3 % |
| - Huile de vaseline Codex | 13 % |
| - Polydiméthylsiloxane ("SILBIONE HUILE 70 047V 300"de Rhône-Poulenc) | 1 % |
| - Triéthanolamine | qsp pH 6-7 |
| - Glycérine | 5 % |
| - Conservateurs | qs |
| - Eau | qsp 100 % |

Les polymères étudiés étaient les suivants :
- SALCARE SC 90 (terpolymère réticulé : Acide méthacrylique / Acrylate d'éthyle / Steareth-10 allyl ether, conforme à l'invention),
- RHEOLATE 5000 (copolymère Acide acrylique / Acétate de vinyle, conforme à l'invention),
- et à titre comparatif le PEMULEN TR-1 de chez Goodrich (copolymère réticulé : Acide acrylique / acrylate d'alkyle en C10-C30 non conforme à l'invention), déja décrit dans des compositions antisolaires et considéré ici comme l'état de la technique le plus proche.

On a réalisé chacune de ces émulsions en dissolvant le filtre dans la phase grasse, puis en ajoutant les émulsionnants dans cette phase grasse portée aux environs de 80°C, et enfin en additionnant sous agitation rapide la phase aqueuse préalablement chauffée à cette même température.

Pour chacune des formulations ainsi préparées, on a ensuite déterminé le facteur de protection solaire (SPF) qui leur était attaché. Celui-ci a été déterminé en utilisant la méthode *in vitro* décrite par B.L. DIFFEY et al. dans J. Soc. Cosmet. Chem. 40-127-133 (1989) ; cette méthode consiste à déterminer les facteurs de protection monochromatiques tous les 5 nm dans une gamme de longueurs d'onde de 290 à 400 nm et à calculer à partir de ceux-ci le facteur de protection solaire selon une équation mathématique donnée.

Les compositions chimiques des formulations et les résultats en facteur de protection moyen obtenus sont rassemblés dans le tableau I donné ci-dessous.

**Tableau I**

| | FORMULATIONS | | | | | |
|---|---|---|---|---|---|---|
| | 1 | 2 | 3 | 4 | 5 | 6 |
| Nanopigments (%) | 5 | 0 | 5 | 0 | 5 | 0 |
| Filtre(%) | 0 | 5 | 0 | 5 | 0 | 5 |
| SALCARE SC90 (% MA) | 1,5 | 1,5 | 0 | 0 | 0 | 0 |
| RHEOLATE 5000 (% MA) | 0 | 0 | 0,5 | 0,5 | 0 | 0 |
| PEMULEN TR-1 (% MA) | 0 | 0 | 0 | 0 | 1 | 1 |
| SPF moyen (écart-type) | **8,5** (1,4) | **8,2** (0,6) | **6,1** (0) | **9,3** (0,4) | **2,8** (0,3) | **6,8** (0,6) |
| | invention | | | | comparatif | |

Ces résultats démontrent clairement les meilleurs propriétés phoprotectrices des compositions conformes à l'invention, que ce soit en présence de filtres organiques ou de nanopigments.

## Revendications

1. Compositions cosmétiques à usage topique, en particulier pour la photoprotection de la peau et/ou des cheveux, du type comprenant, dans un support cosmétiquement acceptable dont la phase continue est aqueuse, un système photoprotecteur capable de filtrer les rayons UV (UV-A et/ou UV-B) et au moins un polymère, et caractérisées par le fait que ledit polymère est choisi au sein des polymères constitués par la répétition (i) d'un motif de formule (I) et (ii) d'un motif de formule (II) et/ou de formule (III) ci-dessous : 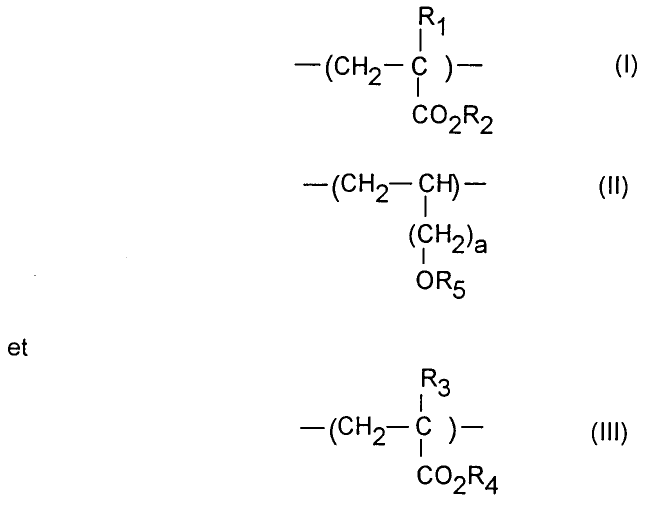 dans lesquelles a est un nombre entier égal à 0 ou 1, R₁, R₂, R₃ et R₄, qui peuvent être identiques ou différents, désignent l'hydrogène ou un radical alkyle en C₁-C₄, et R₅ désigne un radical CH₃CO- ou un radical R₆-(OC₂H₅)_{b}- dans lequel R₆ est un radical alkyle en C₂-C₂₀ et b un nombre entier compris inclusivement entre 1 et 20, étant entendu que lorsque ledit polymère est exempt de motif de formule (II) alors les radicaux R₂ et R₄ ne peuvent représenter simultanément l'hydrogène.

2. Compositions selon la revendication 1, caractérisées par le fait que le ou les polymères sont présents à une teneur comprise entre 0,05 et 15 % en poids par rapport au poids total de la composition.

3. Compositions selon la revendication 2, caractérisées par le fait que ladite teneur est comprise entre 0,1 et 4 % en poids.

4. Compositions selon l'une quelconque des revendications précédentes, caractérisées par le fait que ledit sytème photoprotecteur est constitué par un ou plusieurs filtres organiques absorbeurs d'UV et/ou un ou plusieurs (nano)pigments minéraux.

5. Compositions selon la revendication 4, caractérisées par le fait que lesdits (nano)pigments sont choisis parmi les oxydes de titane, de zinc, de fer, de zirconium, de cérium et leurs mélanges, enrobés ou non.

6. Compositions selon la revendication 5, caractérisées par le fait que l'on utilise des nanopigments.

7. Compositions selon la revendication 6, caractérisées par le fait les nanopigments sont à base d'oxyde de titane, enrobé ou non enrobé.

8. Compositions selon la revendication 7, caractérisées par le fait l'oxyde de titane est sous forme rutile, anatase ou amorphe.

9. Compositions selon l'une quelconque des revendications 4 à 8, caractérisées par le fait que lesdits (nano)pigments sont présents à une teneur comprise entre 0,1 et 30 % en poids par rapport au poids total de la composition.

10. Compositions selon la revendication 9, caractérisées par le fait que ladite teneur est comprise entre 1 et 20 % en poids.

11. Compositions selon la revendication 4, caractérisées par le fait que lesdits filtres organiques sont choisis parmi les dérivés cinnamiques, les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres.

12. Compositions selon la revendication 4 ou 11, caractérisées par le fait que lesdits filtres organiques sont présents à une teneur comprise entre 0,1 et 30 % en poids par rapport au poids total de la composition.

13. Compositions selon la revendication 12, caractérisées par le fait que ladite teneur est comprise entre 0,5 et 25 % en poids.

14. Compositions selon l'une quelconque des revendications précédentes, caractérisées par le fait que la teneur globale en système photoprotecteur n'excède pas 40 % du poids total de la composition.

15. Compositions selon l'une quelconque des revendications précédentes, caractérisées par le fait que le ou les polymères sont choisis parmi les terpolymères réticulés : Acide méthacrylique / Acrylate d'éthyle / Steareth-10 allyl ether ; les copolymères réticulés : Acide acrylique / Acétate de vinyle ; les copolymères réticulés : Acide acrylique / Acrylate d'éthyle.

16. Compositions selon l'une quelconque des revendications précédentes, caractérisées par le fait que ledit support cosmétiquement acceptable se présente sous la forme d'une émulsion de type huile-dans-eau.

17. Compositions selon l'une quelconque des revendications précédentes, caractérisées par le fait qu'elles comprennent en outre au moins un agent de bronzage et/ou de brunissage artificiel de la peau.

18. Compositions selon l'une quelconque des revendications précédentes, caractérisées par le fait qu'elles comprennent en outre au moins un adjuvant choisi parmi les corps gras, les solvants organiques, les épaississants ioniques ou non ioniques, les adoucissants, les antioxydants et notamment les antioxydants ARL, les opacifiants, les stabilisants, les émollients, les silicones , les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants.

19. Compositions selon l'une quelconque des revendications précédentes, caractérisées par le fait qu'il s'agit de compositions protectices de l'épiderme humain ou de compositions antisolaires.

20. Compositions selon l'une quelconque des revendications 1 à 18, caractérisées par le fait qu'il s'agit de compositions de maquillage.

21. Compositions selon l'une quelconque des revendications 1 à 18, caractérisées par le fait qu'il s'agit de compositions destinées à la protection des cheveux contre les rayons ultraviolets.

22. Utilisation des compositions définies à l'une quelconque des revendications précédentes comme, ou pour la fabrication de, compositions cosmétiques pour la protection de la peau et/ou des cheveux contre le rayonnement ultraviolet, en particulier le rayonnement solaire.

23. Procédé de traitement cosmétique pour protéger la peau et/ou les cheveux contre le rayonnement ultraviolet, en particulier le rayonnement solaire, caractérisé en ce qu'il consiste à appliquer sur ceux-ci une quantité efficace d'une composition telle que définie à l'une quelconque des revendications 1 à 21.

## Claims

1. Cosmetic compositions for topical use, especially for photoprotection of the skin and/or hair, of the type comprising a photoprotective system capable of screening out UV (UV-A and/or UV-B) rays and at least one polymer in a cosmetically acceptable vehicle in which the continuous phase is aqueous, and characterized in that the said polymer is chosen from polymers consisting of the repetition (i) of a unit of formula (I) and (ii) of a unit of formula (II) and/or of formula (III) below: 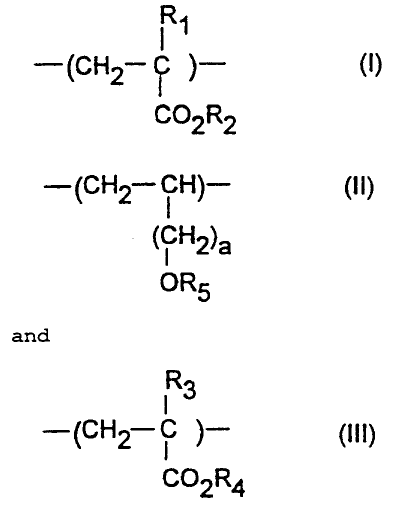 in which a is an integer equal to 0 or 1, R₁, R₂, R₃ and R₄, which may be identical or different, denote hydrogen or a C₁-C₄ alkyl radical, and R₅ denotes a CH₃CO- radical or a radical R₆-(OC₂H₅)_{b}- in which R₆ is a C₂-C₂₀ alkyl radical and b an integer between 1 and 20 inclusive, on the understanding that when the said polymer lacks a unit of formula (II), the radicals R₂ and R₄ cannot then simultaneously represent hydrogen.

2. Compositions according to Claim 1, characterized in that the polymer or polymers is/are present at a content of between 0.05 and 15 % by weight relative to the total weight of the composition.

3. Compositions according to Claim 2, characterized in that the said content is between 0.1 and 4 % by weight.

4. Compositions according to any one of the preceding claims, characterized in that the said photoprotective system consists of one or more UV-absorbing organic screening agents and/or one or more inorganic (nano)pigments.

5. Compositions according to Claim 4, characterized in that the said (nano)pigments are chosen from titanium, zinc, iron, zirconium and cerium oxides and mixtures thereof, coated or otherwise.

6. Compositions according to Claim 5, characterized in that nanopigments are used.

7. Compositions according to Claim 6, characterized in that the nanopigments are based on titanium oxide, coated or uncoated.

8. Compositions according to Claim 7, characterized in that the titanium oxide is in rutile, anatase or amorphous form.

9. Compositions according to any one of Claims 4 to 8, characterized in that the said (nano)pigments are present at a content of between 0.1 and 30 % by weight relative to the total weight of the composition.

10. Compositions according to Claim 9, characterized in that the said content is between 1 and 20 % by weight.

11. Compositions according to Claim 4, characterized in that the said organic screening agents are chosen from cinnamic derivatives, salicylic derivatives, camphor derivatives, triazine derivatives, benzophenone derivatives, dibenzoylmethane derivatives, β,β-diphenylacrylate derivatives, p-aminobenzoic acid derivatives, screening polymers and screening silicones.

12. Compositions according to Claim 4 or 11, characterized in that the said organic screening agents are present at a content of between 0.1 and 30 % by weight relative to the total weight of the composition.

13. Compositions according to Claim 12, characterized in that the said content is between 0.5 and 25 % by weight.

14. Compositions according to any one of the preceding claims, characterized in that the overall content of the photoprotective system does not exceed 40 % of the total weight of the composition.

15. Compositions according to any one of the preceding claims, characterized in that the polymer or polymers is/are chosen from crosslinked terpolymers: methacrylic acid/ethyl acrylate/steareth-10 allyl ether; crosslinked copolymers: acrylic acid/vinyl acetate; and crosslinked copolymers: acrylic acid/ethyl acrylate.

16. Compositions according to any one of the preceding claims, characterized in that the said cosmetically acceptable vehicle takes the form of an oil-in-water type emulsion.

17. Compositions according to any one of the preceding claims, characterized in that they comprise, in addition, at least one agent for artificially bronzing and/or tanning the skin.

18. Compositions according to any one of the preceding claims, characterized in that they comprise, in addition, at least one adjuvant chosen from fats, organic solvents, ionic or nonionic thickeners, demulcents, antioxidants, and in particular anti-free-radical antioxidants, opacifiers, stabilizers, emollients, silicones, α-hydroxy acids, antifoaming agents, hydrating agents, vitamins, perfumes, preservatives, surfactants, fillers, sequestering agents, polymers, propellants, alkalinizing or acidifying agents and colorants.

19. Compositions according to any one of the preceding claims, characterized in that they are compositions for protecting the human epidermis or antisun compositions.

20. Compositions according to any one of Claims 1 to 18, characterized in that they are make-up compositions.

21. Compositions according to any one of Claims 1 to 18, characterized in that they are compositions intended for the protection of hair against ultraviolet rays.

22. Use of the compositions defined in any one of the preceding claims as, or for the manufacture of, cosmetic compositions for the protection of the skin and/or hair against ultraviolet radiation, especially solar radiation.

23. Cosmetic treatment process for protecting the skin and/or hair against ultraviolet radiation, especially solar radiation, characterized in that it consists in applying an effective amount of a composition as defined in any one of Claims 1 to 21 to the skin and/or hair.

## Patentansprüche

1. Kosmetische Zusammensetzungen zur topischen Anwendung und insbesondere zum Lichtschutz der Haut und/oder der Haare, die in einem kosmetisch akzeptablen Träger, dessen kontinuierliche Phase wäßrig ist, ein Lichtschutzsystem, das UV-(UV-A- und/oder UV-B-)Strahlung filtern kann, und mindestens ein Polymer enthalten und die dadurch gekennzeichnet sind, daß das Polymer unter den Polymeren ausgewählt ist, die aus wiederkehrenden Einheiten (i) aus einer Einheit der Formel I und wiederkehrenden Einheiten (ii) aus einer Einheit der Formel II und/oder der Formel III bestehen: 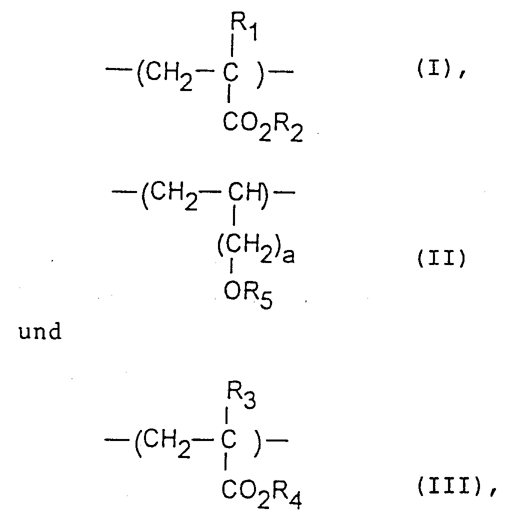 worin bedeuten:
a Null oder 1,
R₁, R₂, R₃ und R₄, die gleich oder voneinander verschieden sein können, Wasserstoff oder eine C₁₋₄-Alkylgruppe,
R₅ eine Gruppe CH₃CO- oder eine Gruppe R₆-(OC₂H₅)_{b}-,
worin R₆ eine C₂₋₂₀-Alkylgruppe und b eine ganze Zahl von 1 bis 20 bedeutet,
mit der Maßgabe, daß die Gruppen R₂ und R₄ nicht gleichzeitig Wasserstoff bedeuten, wenn das Polymer keine Einheiten der Formel II enthält.

2. Zusammensetzungen nach Anspruch 1, dadurch gekennzeichnet, daß das oder die Polymeren in einem Anteil im Bereich von 0,05 bis 15 Gew.-%, bezogen auf das Gesamtgewicht der Zusammensetzung, vorliegen.

3. Zusammensetzungen nach Anspruch 2, dadurch gekennzeichnet, daß der Anteil im Bereich von 0,1 bis 4 Gew.-% liegt.

4. Zusammensetzungen nach einem der vorhergehenden Ansprüche, dadurch gekennzeichnet, daß das Lichtschutzsystem aus einem oder mehreren organischen Filtern, die UV-Strahlung absorbieren, und/oder einem oder mehreren anorganischen (Nano)pigmenten besteht.

5. Zusammensetzungen nach Anspruch 4, dadurch gekennzeichnet, daß die (Nano)pigmente, die ggf. umhüllt sind, unter Titanoxid, Zinkoxid, Eisenoxid, Zirconiumoxid und Ceroxid sowie deren Gemischen ausgewählt sind.

6. Zusammensetzungen nach Anspruch 5, dadurch gekennzeichnet, daß Nanopigmente verwendet sind.

7. Zusammensetzungen nach Anspruch 6, dadurch gekennzeichnet, daß die Nanopigmente auf der Basis von ggf. umhülltem Titanoxid vorliegen.

8. Zusammensetzungen nach Anspruch 7, dadurch gekennzeichnet, daß das Titanoxid in Form von Rutil oder Anatas oder in amorpher Form vorliegt.

9. Zusammensetzungen nach einem der Ansprüche 4 bis 8, dadurch gekennzeichnet, daß die (Nano)pigmente in einem Anteil im Bereich von 0,1 bis 30 Gew.-%, bezogen auf das Gesamtgewicht der Zusammensetzung, vorliegen.

10. Zusammensetzungen nach Anspruch 9, dadurch gekennzeichnet, daß der Anteil im Bereich von 1 bis 20 Gew.-% liegt.

11. Zusammensetzungen nach Anspruch 4, dadurch gekennzeichnet, daß die organischen Filter unter Zimtsäurederivaten, Salicylsäurederivaten, Campherderivaten, Triazinderivaten, Benzophenonderivaten, Dibenzoylmethanderivaten, β,β-Diphenylacrylatderivaten, p-Aminobenzoesäurederivaten, polymeren Filtern und Siliconfiltern ausgewählt sind.

12. Zusammensetzungen nach Anspruch 4 oder 11, dadurch gekennzeichnet, daß die organischen Filter in einem Anteil im Bereich von 0,1 bis 30 Gew.-%, bezogen auf das Gesamtgewicht der Zusammensetzung, vorliegen.

13. Zusammensetzungen nach Anspruch 12, dadurch gekennzeichnet, daß der Anteil im Bereich von 0,5 bis 25 Gew.-% liegt.

14. Zusammensetzungen nach einem der vorhergehenden Ansprüche, dadurch gekennzeichnet, daß der Gesamtgehalt des Lichtschutzsystems höchstens 40 % des Gesamtgewichts der Zusammensetzung beträgt.

15. Zusammensetzungen nach einem der vorhergehenden Ansprüche, dadurch gekennzeichnet, daß das oder die Polymere unter vernetzten Methacrylsäure-Ethylacrylat-Steareth-10-allylether-Terpolymeren, vernetzten Acrylsäure-Vinylacetat-Copolymeren und vernetzten Acrylsäure-Ethylacrylat-Copolymeren ausgewählt sind.

16. Zusammensetzungen nach einem der vorhergehenden Ansprüche, dadurch gekennzeichnet, daß der kosmetisch akzeptable Träger in Form einer Öl-in-Wasser-Emulsion vorliegt.

17. Zusammensetzungen nach einem der vorhergehenden Ansprüche, dadurch gekennzeichnet, daß sie ferner mindestens ein Bräunungsmittel und/oder ein Mittel zur künstlichen Hautbräunung enthalten.

18. Zusammensetzungen nach einem der vorhergehenden Ansprüche, dadurch gekennzeichnet, daß sie ferner mindestens einen Zusatzstoff enthalten, der unter Fettsubstanzen, organischen Lösungsmitteln, ionischen oder nichtionischen Verdickungsmitteln, reizlindernden Mitteln, Antioxidationsmitteln und insbesondere Mitteln gegen freie Radikale, Trübungsmitteln, Stabilisatoren, Softenern, Siliconen, α-Hydroxysäuren, Antischaummitteln, Feuchthaltemitteln, Vitaminen, Parfums, Konservierungsmitteln, grenzflächenaktiven Stoffen, Füllstoffen, Maskierungsmitteln, Polymeren, Treibmitteln, alkalisch oder sauer machenden Mitteln und Färbemitteln ausgewählt sind.

19. Zusammensetzungen nach einem der vorhergehenden Ansprüche, dadurch gekennzeichnet, daß es sich um Zusammensetzungen zum Schutz der menschlichen Epidermis oder um Sonnenschutzmittel handelt.

20. Zusammensetzungen nach einem der Ansprüche 1 bis 18, dadurch gekennzeichnet, daß es sich um eine Zusammensetzung zum Schminken handelt.

21. Zusammensetzungen nach einem der Ansprüche 1 bis 18, dadurch gekennzeichnet, daß es sich um Zusammensetzungen handelt, die zum Schutz der Haare gegen UV-Strahlung bestimmt sind.

22. Verwendung der Zusammensetzungen nach einem der vorhergehenden Ansprüche als kosmetische Zusammensetzungen oder für die Herstellung von kosmetischen Zusammensetzungen zum Schutz der Haut und/oder der Haare gegen UV-Strahlung und insbesondere gegen Sonnenlicht.

23. Verfahren zur kosmetischen Behandlung zum Schutz der Haut und/oder der Haare gegen UV-Strahlung und insbesondere gegen Sonnenlicht, dadurch gekennzeichnet, daß es darin besteht, eine wirksame Menge einer in den Ansprüchen 1 bis 21 definierten Zusammensetzung auf die Haut und/oder das Haar aufzutragen.
